Europäisches Patentamt

European Patent Office

Office européen des brevets

(11) Veröffentlichungsnummer: **0 235 553 B1**

# (12) EUROPÄISCHE PATENTSCHRIFT

(45) Veröffentlichungstag der Patentschrift: **18.12.91**

(21) Anmeldenummer: **87101007.0**

(22) Anmeldetag: **24.01.87**

(51) Int. Cl.⁵: **C07D 201/08, B01J 23/78,
B01J 25/00**

(54) **Verfahren zur Herstellung von Epsilon-Caprolactam.**

(30) Priorität: **28.01.86 DE 3602375**

(43) Veröffentlichungstag der Anmeldung:
**09.09.87 Patentblatt 87/37**

(45) Bekanntmachung des Hinweises auf die
Patenterteilung:
**18.12.91 Patentblatt 91/51**

(84) Benannte Vertragsstaaten:
**BE CH DE FR GB IT LI NL**

(56) Entgegenhaltungen:
**DE-C- 952 442
GB-A- 1 191 539**

(73) Patentinhaber: **BASF Aktiengesellschaft
Carl-Bosch-Strasse 38
W-6700 Ludwigshafen(DE)**

(72) Erfinder: **Hutmacher, Hans-Martin, Dr.
Ruedigerstrasse 70
W-6700 Ludwigshafen(DE)**
Erfinder: **Merger, Franz, Dr.
Max-Slevogt-Strasse 25
W-6710 Frankenthal(DE)**
Erfinder: **Broecker, Franz Josef, Dr.
Schwanthaler Allee 20
W-6700 Ludwigshafen(DE)**
Erfinder: **Fischer, Rolf, Dr.
Bergstrasse 98
W-6900 Heidelberg(DE)**
Erfinder: **Vagt, Uwe, Dr.
Paul-Neumann-Strasse 44
W-6720 Speyer(DE)**
Erfinder: **Harder, Wolfgang, Dr.
Bergwaldstrasse 16
W-6940 Weinheim(DE)**
Erfinder: **Priester, Claus-Ulrich, Dr.
Wiesenstrasse 18
W-6701 Meckenheim(DE)**

**Beschreibung**

Gegenstand der Erfindung ist ein Verfahren zur Herstellung von ε-Caprolactam aus 5-Formylvaleriansäureestern.

In der GB-PS 1 191 539 wird ein Verfahren zur Herstellung von ε-Caprolactam beschrieben, bei dem man 5-Formylvaleriansäureester mit Wasserstoff, Ammoniak und Wasserdampf in der Gasphase bei 260 °C unter Mitverwendung von Kupferkatalysatoren umsetzt. Schwierigkeiten bestehen jedoch hinsichtlich der schlechten Verdampfbarkeit des thermolabilen 5-Formylvaleriansäureesters und den ungenügenden Katalysatorstandzeiten. Aus der japanischen Patentveröffentlichung 29148/1968 ist auch bekannt, daß man 5-Formylvaleriansäureester mit Ammoniak in Gegenwart von Wasser bei einer Temperatur von 230 °C und unter einem Druck von 150 bar sowie in Gegenwart von Raney-Nickel in flüssiger Phase umsetzt. Dieses Verfahren hat den Nachteil, daß es bei der technischen Ausführung mit sehr schwankenden Ausbeuten verläuft. Aus der DE-C- 952 442 ist bekannt, daß man Formylvaleriansäureester mit Wasserstoff und Ammoniak in wasserfreiem Dioxan in Gegenwart von Raney-Kobalt, beginnend bei 60 °C und ansteigend bis auf 130 °C, innerhalb von 7 Stunden umsetzt. Hierbei erhält man Aminocapronsäureester in einer Ausbeute von 24 % und Caprolactam in einer Ausbeute von 40 %.

Es war deshalb die technische Aufgabe gestellt, ein Verfahren zur Herstellung von ε–Caprolactam, ausgehend von 5 - Formylvaleriansäureestern zur Verfügung zu stellen, das mit hohen Ausbeuten verläuft und bei dem möglichst wenig Nebenprodukt gebildet werden.

Diese Aufgabe wird gelöst in einem Verfahren zur Herstellung von ε-Caprolactam durch Umsetzen von 5-Formylvaleriansäureestern mit Ammoniak im Überschuß und Wasserstoff in Gegenwart von Hydrierkatalysatoren unter Mitverwendung von Lösungsmitteln bei erhöter Temperatur unter erhöhtem Druck in flüssiger Phase, dadurch gekennzeichnet, daß man

a) 5-Formylvaleriansäureester mit überschüssigen Ammoniak und Wasserstoff unter Mitverwendung von Alkanolen als Lösungsmittel in Gegenwart von Nickel- oder Kobalt-Katalysatoren unter einem Wasserstoffpartialdruck in flüssiger Phase bei einer Temperatur von 40 bis 130 °C umsetzt,
b) aus dem Reaktionsgemisch überschüssigen Ammoniak bis auf einen Gehalt von 0,1 bis 2 Gew.% und Wasserstoff abtrennt,
c) das so erhaltene Reaktionsgemisch bei einer Temperatur von 50 bis 250 °C mit der 1- bis 20-fachen Gewichtsmenge Wasser, bezogen auf den Gesamtgehalt an 6-Aminocapronsäureester und Caprolactam, unter gleichzeitigem Abtrennen von Alkanolen umsetzt, und
d) das so erhaltene Reaktionsgemisch auf eine Temperatur von 150 bis 370 °C erhitzt und ε-Caprolactam gewinnt.

Das neue Verfahren hat den Vorteil, daß es mit hohen Ausbeuten verläuft und wenig Nebenprodukte gebildet werden.

Bevorzugte 5-Formylvaleriansäureester sind 5-Formylvaleriansäurealkylester, insbesondere solche von $C_1$- bis $C_4$-Alkanolen wie Methyl-, Ethyl-, Propyl-, Isopropyl- oder n-Butylester. Geeignete Ausgangsverbindungen sind demnach 5-Formylvaleriansäuremethylester, 5-Formylvaleriansäurepropylester, 5-Formylvaleriansäureisopropylester, 5-Formylvaleriansäureethylester oder 5-Formylvaleriansäure-n-butylester. Besondere technische Bedeutung hat 5-Formylvaleriansäuremethylester erlangt.

Die Umsetzung in der Stufe a) wird unter Mitverwendung von Alkanolen als Lösungsmittel durchgeführt. Vorteilhaft verwendet man Alkanole, die der Alkoholkomponente des 5-Formylvaleriansäureesters entsprechen. Demzufolge sind bevorzugte Lösungsmittel Methanol, Ethanol, Propanol, Isopropanol oder n-Butanol. Besonders bevorzugt ist die Kombination 5-Formylvaleriansäuremethylester/Methanol. Vorteilhaft werden die 5-Formylvaleriansäureester als 1 bis 50 gew.-%ige, vorteilhaft 2 bis 35 gew.-%ige, insbesondere 5 bis 25 gew.-%ige Lösung in den genannten Lösungsmitteln eingesetzt.

Bei der Umsetzung wendet man Ammoniak im Überschuß an. Vorteilhaft verwendet man je Mol 5-Formylvaleriansäureester, 2 bis 50 Mol Ammoniak. Besonders gute Ergebnisse erhält man, wenn man je Mol 5-Formylvaleriansäureester 5 bis 30 Mol, insbesondere 10 bis 25 Mol, Ammoniak anwendet.

Die Umsetzung wird in flüssiger Phase bei einer Temperatur von 40 bis 130 °C, vorteilhaft von 40 bis 95 °C, insbesondere 60 bis 90 °C durchgeführt.

Vorteilhaft wendet man je Mol 5-Formylvaleriansäureester 1 bis 20 Mol Wasserstoff an. Hierbei hält man einen Wasserstoffpartialdruck von 5 bis 1000 bar, vorzugsweise 20 bis 500 bar, insbesondere 50 bis 200 bar, aufrecht.

Die Umsetzung wird in Gegenwart von Nickel- oder Kobalt-Katalysatoren durchgeführt. Die Katalysatormetalle können als Vollkatalysatoren, z.B. in feiner Verteilung wie Raney-Nickel oder Raney-Kobalt, in Suspensionsfahrweise oder in magnetischer Fixierung, als Mischkatalysatoren oder auf Trägern niedergeschlagen angewandt werden. Geeignete Träger sind beispielsweise Aluminiumoxid, Kieselgel oder Magnesiumsilikate.

Gut geeignet sind auch Skelett-Katalysatoren. Die katalytisch aktiven Metalle werden besonders vorteilhaft in feiner Verteilung angewandt. Deshalb haben sich Skelett-Katalysatoren als besonders geeignet erwiesen.

Besonders bevorzugt wendet man Katalysatoren, die durch Kalzinieren von Verbindungen der Formel I

$$[(Mg_aNi(II)_bCo(II)_cAl_2]CO_3(OH)_{16} \times 4\, H_2O$$

in der a für ganze oder Dezimalzahlen von 0 bis 4 steht und b und c für ganze Zahlen oder Dezimalzahlen von 0 bis 6 stehen, mit der Maßgabe, daß 2 (a + b + c) = 12 ist, bei einer Temperatur von 200 bis 600°C und anschließende Reduktion mit Wasserstoff bei erhöhter Temperatur, z.B. von 350 bis 400°C, hergestellt wurden. Besonders bewährt haben sich Katalysatoren, die erhalten wurden durch Kalzinieren und Reduzieren von Verbindungen der folgenden Formeln

$$Ni_6Al_2(OH)_{16}CO_3 \times 4\, H_2O$$
$$Ni_5MgAl_2(OH)_{16}CO_3 \times 4\, H_2O$$
$$Co_6Al_2(OH)_{16}CO_3 \times 4\, H_2O$$
$$Co_5MgAl_2(OH)_{16}CO_3 \times 4\, H_2O.$$

Die Verbindungen der Formel I erhält man beispielsweise wie folgt:
Nickel, Aluminium, Kobalt und Magnesium werden in Form ihrer wasserlöslichen Salze wie Chloride, Sulfate oder vorzugsweise Nitrate, gemeinsam in Wasser gelöst und zwar in einem Mengenverhältnis, das der gewünschten Zusammensetzung des Katalysators möglichst nahe kommt und in seiner Stöchiometrie der Formel I gehorcht.

Die Metallsalzlösung soll insgesamt etwa 0,5 bis 3 molar, vorzugsweise 1,0 bis 2 molar an Metallionen sein. Sie wird auf Temperaturen von 50 bis 100°C, vorzugsweise bis 100°C, erhitzt und innerhalb von 0,5 bis 10, vorzugsweise bis 3 Minuten, mit einer äquivalenten Menge oder bevorzugt einem geringen Überschuß einer auf 50 bis 100°C, vorzugsweise 80 bis 100°C erhitzten, 1 bis 3, vorzugsweise 1,5 bis 2,5 molaren Lösung eines Alkalibicarbonats vereinigt. Vorteilhaft arbeitet man mit einem Überschuß an Alkalibicarbonat, der bis zu 20 Gew.-%, vorzugsweise 0,5 bis 3 Gew.-%, bezogen auf die theoretische Menge des Bicarbonats, beträgt. Nach der Zugabe der Metallsalzlösung wird zweckmäßig noch 10 bis 30 Minuten, vorzugsweise 15 bis 25 Minuten gerührt und dann der entstehende Niederschlag abfiltriert, mit Wasser gewaschen und bei einer Temperatur von 50 bis 200°C, vorzugsweise 100 bis 160°C getrocknet. Die basischen Carbonate werden in nahezu quantitativen Ausbeuten erhalten. Als Alkalibicarbonate kommen insbesondere Natriumbicarbonat oder Kaliumbicarbonat in Betracht. Es ist aber auch möglich, Ammoniumbicarbonat bei der Fällung zu verwenden. Selbstverständlich können auch Mischungen der genannten Bicarbonate verwendet werden. Außerdem ist es möglich, die Fällung der Metallionen mit Lösungen von Alkalicarbonaten wie Natriumcarbonat und/oder Kaliumcarbonat vorzunehmen, wenn man während des Fällens Kohlendioxid in die die vorgelegte Alkalicarbonatlösung einleitet, was aber im Endeffekt auf eine Fällung mit Bicarbonat hinausläuft. Vorteilhaft wendet man beim Kalzinieren eine Temperatur von 250 bis 400°C für eine Dauer von z.B. 5 bis 40 Stunden, insbesondere 15 bis 30 Stunden an. Vor der eigentlichen Verwendung des Katalysators wird dieser mit Wasserstoff vorteilhaft bei einer Temperatur von 180 bis 500°C, vorzugsweise 250 bis 450°C, innerhalb von 5 bis 100 Stunden, vorteilhaft 10 bis 100 Stunden, reduziert.

Andere bevorzugte Katalysatoren sind Nickelkatalysatoren, die Nickel in fein verteilter Form auf Träger, insbesonder Magnesiumsilikat, aufgebracht enthalten. Vorteilhaft haben solche Katalysatoren einen Gehalt an Nickel von 30 bis 60 Gew.-%, bezogen auf die gesamte Katalysatormasse einschließlich Träger. Solche Katalysatoren werden beispielsweise in der DE-PS 15 45 428 beschrieben.

Bevorzugt wird Raney-Nickel oder Raney-Kobalt als Katalysator verwendet, wobei man in Suspensionsfahrweise arbeitet oder den Katalysator in der Reaktionszone an Permanentmagneten oder an Weicheisenkörpern in einem magnetischen bzw. elektromagnetischen Feld fixiert. Solche Magnete sind z.B. in Form von Stäben in der Reaktionszone angeordnet.

Für die Umsetzung hat es sich weiterhin als vorteilhaft erwiesen, eine Verweilzeit von 1 bis 30 Minuten sowie Katalysatorbelastungen von 0,2 bis 2,0 kg 5-Formylvaleriansäureester je Liter Katalysator und Stunde einzuhalten.

Die Umsetzung kann diskontinuierlich, z.B. in einem Hochdruckgefäß, durchgeführt werden. Vorzugsweise führt man die Umsetzung kontinuierlich, z.B. in druckfesten Rührbehältern, z.B. einer Rührbehälterkaskade durch. Es hat sich als vorteilhaft erwiesen, eine Rückvermischung während der Umsetzung zu vermeiden. Besonders bewährt haben sich deshalb Rohrreaktoren, in denen man die alkoholische Lösung von 5-Formylvaleriansäureester und Ammoniak über ein fest angeordnetes Katalysatorbett leitet. Besonders bewährt hat sich hierbei die Sumpffahrweise.

Als Reaktionsaustrag aus der Stufe a erhält man nach dem Entspannen, bei dem der Wasserstoff entfernt wird, Gemische aus 6-Aminocarbonsäureestern, dem mitverwendeten Alkanol, überschüssigem Ammoniak und untergeordneten Mengen an ε-Caprolactam.

Bei der Verwendung von 5-Formylvaleriansäuremethylester und Methanol als Lösungsmittel erhält man ein Reaktionsgemisch aus 6-Aminocapronsäuremethylester in Methanol mit einem Anteil von 1 bis 10 Mol.-% an ε-Caprolactam, bezogen auf 6-Aminocapronsäuremethylester sowie Ammoniak.

In einer zweiten Stufe b wird das überschüssige Ammoniak sowie gelöster Wasserstoff aus dem Reaktionsgemisch entfernt. Dies erfolgt beispielsweise durch Abdestillieren oder durch Strippen mit Inertgas. Die anfallenden Mengen an Ammoniak sowie überschüssiger Wasserstoff werden vorteilhaft wieder in die Stufe A zurückgeführt. Man hält einen Ammoniakgehalt in der der weiter zu verwendenden Lösung Von 0,1 bis 2 Gew.-%, insbesondere von 0,1 bis 1 Gew.-% ein.

Das so erhaltene Reaktionsgemisch, das im wesentlichen 6-Aminocapronsäure, Alkanole und geringe Mengen Ammoniak und ε-Caprolactam enthält, wird anschließend mit Wasser unter gleichzeitigem Abtrennen des Alkanols bei erhöhter Temperatur umgesetzt (Schritt c).

Man verwendet die 1- bis 20-, insbesondere 3- bis 10-fache Gewichtsmenge Wasser bezogen auf den Gesamtgehalt an 6-Aminocapronsäureester und ε-Caprolactam.

Geeignete Reaktionstemperaturen sind von 50 bis 250°C, insbesondere 80 bis 150°C. Die Reaktion wird je nach Reaktionstemperatur bei Normaldruck oder unter Überdruck z.B. bis zu 5 bar diskontinuierlich oder kontinuierlich durchgeführt. Bevorzugt arbeitet man kontinuierlich, z.B. in Rührreaktoren oder Rührreaktorkaskaden, die mit geeigneten Destillationseinrichtungen zur Abtrennung des Alkanols ausgerüstet sind.

Die abgetrennten Alkanole werden zweckmäßig zweckmäßig in die Hydrierstufe (a) zurückgeführt.

Man strebt vorteilhaft Umsätze des 6-Aminocapronsäureesters von ≥ 90 % insbesondere ≥ 95 % an.

Es hat sich auch bewährt, die Stufen b und c zusammenzufassen und in einer Kolonne zunächst Ammoniak abzudestillieren und unter Mitverwendung von Wasser und gleichzeitigem Abdestillieren von Alkanolen, die Umsetzung durchzuführen. Hierbei führt man das Reaktionsgemisch aus der Stufe a nach dem Entspannen zweckmäßig dem mittleren Teil einer Kolonnie und dem Sumpf der Kolonne Wasser zu. Ammoniak und Alkanole werden abdestilliert, während als Sumpf eine wäßrige Lösung von 6-Aminocapronsäure anfällt.

In (d) wird 6-Aminocapronsäure die als Reaktionsaustrag der vorausgegangenen Hydrolysestufe (c) erhalten wird zu ε-Caprolactam cyclokondensiert.

Bevorzugt verwendet man die Reaktionsausträge aus der Stufe c direkt. Man kann die Reaktionsausträge jedoch falls erforderlich aufkonzentrieren z.B. durch Abdestillieren eines Teiles des Wassers oder durch Zusatz von Wasser verdünnen.

Bevorzugt werden für die Cyclokondensation Lösungen verwendet, die 1 bis 20 Gew.-%, insbesondere 2 bis 10 Gew.-% 6-Aminocapronsäure enthalten. Die Umsetzung wird bei Temperaturen von 150 bis 370°C, insbesondere 250 bis 330°C, im allgemeinen unter dem sich einstellenden Druck des Reaktionsgemisches oder unter Überdruck, diskontinuierlich oder kontinuierlich, z.B. in einem Rohrreaktor, durchgeführt. Die mittleren Verweilzeiten betragen hierbei 0,1 bis 2 Stunden.

Die Abtrennung des gebildeten ε-Caprolactams wird durch Destillation oder bevorzugt extraktiv, z.B. mit Extraktionsmitteln wie Methylenchlorid, Chloroform, Cyclohexan oder Trichlorethylen durchgeführt.

Beispiel

Stufe a)

Ein vertikaler Rohrreaktor (Durchmesser 16 mm, Füllhöhe 25 cm, ölbeheizter Doppelmantel) wurde mit 50 ml eines Nickelkatalysators mit 55 Gew.-% Nickeloxid in feiner Verteilung auf Magnesiumsilikat in Form von Strängen von 1,5 mm Durchmesser gefüllt. Der Katalysator wurde in 18 Stunden unter schrittweisem Anheben der Temperatur von 60 auf 330°C unter Steigern des Wasserstoffgehalts in dem zur Reduktion benutzten Stickstoff-Wasserstoff-Gemisch von 5 auf 50 % reduziert.

Danach wurden unter gleichzeitigem Durchleiten von Wasserstoff bei 80°C/100 bar stündlich von unten nach oben 198,9 g einer 10,0 %igen methanolischen 5-Formylvaleriansäuremethylester-Lösung und 36,5 g flüssiges Ammoniak durch den Reaktor gepumpt. Vom Kopf des Reaktors gelangte das Reaktionsgemisch über einen Kühler in einen Abscheider, aus dem das Reaktionsgemisch und stündlich 26,2 l Abgas ausgeschleust wurden.

Das Reaktionsgemisch enthielt nach quantitativer gaschromatographischer Analyse 7,2 Gew.-% 6-Aminocapronsäuremethylester und 0,12 Gew.-% ε-Caprolactam.

Stufen b) und c):

In einem mit einer aufgesetzten Füllkörperkolonne (80 cm hoch, V₂A-Maschendrahtringe 5 mm Ø) versehenen Rührkolben wurden 500 g Wasser zum Sieden erhitzt (100°C). Während 6 Stunden wurde der Reaktionsaustrag aus der Stufe a in die Mitte der Kolonne eingeleitet und dabei über Kopf Ammoniak, Methanol und etwas Wasser abgetrennt (Siedegranze 65°C). Während der letzten 4 Stunden wurden in den Kolben zusätzlich kontinuierlich 995 g Wasser zugepumpt und gleichzeitig mit einer zweiten Pumpe durch Abpumpen von Reaktionsgemisch die Füllhöhe konstant gehalten. Nach Abbruch der Versuche waren insgesamt 1540,2 g Reaktionsgemisch (Hydrolyseprodukt) erhalten worden (abgepumpte einschließlich im Sumpf der Kolonne vorhandene Lösung).

Stufe d):

Die aus der vorhergehenden Stufe erhaltene Lösung wurde danach innerhalb 11 Stunden kontinuierlich bei 110 bar durch einen mit einem Ölthermostaten auf 330°C temperierten Rohrreaktor (Rohrschlange) von 8,71 m Länge und 3,17 mm Durchmesser gepumpt. Am Rohrreaktorausgang wurde das Reaktionsgemisch auf Raumtemperatur abgekühlt und auf Normaldruck entspannt. Im Anschluß wurde der Reaktor eine Stunde durch Durchpumpen von Wasser nachgespült. Insgesamt wurden dabei 1661,1 g Austrag erhalten, der nach quantitativer Analyse durch Hochdruckflüssigkeitschromatographie 4,6 Gew.-% ε-Caprolactam und 0,18 Gew.-% 6-Aminocapronsäure enthielt, entsprechend einer Ausbeute von 81,6 % ε-Caprolactam und 2,8 % 6-Aminocapronsäure, bezogen auf den in die Stufe a eingesetzten 5-Formylvaleriansäuremethylester.

Beispiel 2

Man verfährt wie in Beispiel 1, führt die Stufe a jedoch wie folgt durch:
In einem vertikalen Rohrreaktor (Durchmeser: 14 mm, Länge: 450 mm), ist ein Stab mit einem Durchmesser von 9 mm zentral angeordnet, auf dem Permanentmagnete (mit der Feldstärke 500 Gauß) befestigt sind. Die Magnete werden nur 11,0 g Raney-Nickel beladen, indem man eine Suspension von Raney-Nickel in Wasser von unten nach oben durch den Reaktor leitet.
Danach werden unter gleichzeitigem Durchleiten von 8,7 1 Wasserstoff bei 76°C und 80 bar stündlich von unten nach oben 77,3 g einer 12,0 gew.-%igen methanolischen 5-Formylvaleriansäuremethylester-Lösung und 24 ml flüssiges Ammoniak durch den Reaktor gepumpt. Vom Kopf des Reaktors gelangt das Reaktionsgemisch über ein Druckhalteventil in die Stufe 3.
Die Stufen b, c und d werden wie in Beispiel 1 beschrieben durchgeführt. Man erhält eine Ausbeute an ε-Caprolactam von 85 %.

**Patentansprüche**

1. Verfahren zur Herstellung von ε-Caprolactam durch Umsetzung Von 5-Formylvaleriansäureestern mit überschüssigen Ammoniak und Wasserstoff in Gegenwart von Hydrierkatalysatoren unter Mitverwendung von Lösungsmitteln bei erhöhter Temperatur und unter erhöhtem Druck in flüssiger Phase, dadurch gekennzeichnet, daß man

a) 5-Formylvaleriansäureester mit überschüssigem Ammoniak und Wasserstoff unter Mitverwendung von Alkanolen als Lösungsmittel in Gegenwart von Nickel- oder Kobalt-Katalysatoren unter einem Wasserstoffpartialdruck von 5 bis 1000 bei einer Temperatur von 40 bis 130°C umsetzt,

b) aus dem erhaltenen Reaktionsgemisch Ammoniak bis auf einen Gehalt von 0,1 bis 2 Gew.% und Wasserstoff entfernt,

c) das so erhaltene Reaktionsgemisch bei einer Temperatur von 50 bis 250°C mit der 1- bis 20-fachen Gewichtsmenge Wasser, bezogen auf den Gesamtgehalt an 6-Aminocapronsäureester und ε-Caprolactam, unter gleichzeitigem Abtrennen von Alkanolen umsetzt und

d) das so erhaltene Reaktionsgemisch auf eine Temperatur von 150 bis 370°C erhitzt und anschließend ε-Caprolactam gewinnt.

2. Verfahren nach Anspruch 1, dadurch gekennzeichnet, daß man Katalysatoren verwendet, die durch Kalzinieren von Verbindungen der Formel I

$$[(Mg_aNi(II)_bCo(II)_c)Al_2]CO_3(OH)_{16} \times 4\ H_2O$$

in der a für ganze oder Dezimalzahlen von 0 bis 4 steht und b und c für ganze oder Dezimalzahlen von 0 bis 6 stehen, mit der Maßgabe, daß 2 × (a + b + c) = 12 ist, bei Temperaturen von 200 bis 600°C und anschließende Reduktion mit Wasserstoff bei erhöhter Temperatur hergestellt worden sind.

3. Verfahren nach Anspruch 1, dadurch gekennzeichnet, daß man Nickel-Katalysatoren verwendet, die 30 bis 60 Gew.-% Nickel in fein verteilter Form auf Magnesiumsilikat niedergeschlagen enthalten.

4. Verfahren nach den Ansprüchen 1 bis 3, dadurch gekennzeichnet, daß man 5-Formylvaleriansäureester in Lösung mit Alkanolen und Ammoniak in Sumpffahrweise über ein fest angeordnetes Katalysatorbett leitet.

5. Verfahren nach Anspruch 1, dadurch gekennzeichnet, daß man Raney-Nickel oder Raney-Kobalt in Suspensionsfahrweise verwendet.

6. Verfahren nach Anspruch 1 und 5, dadurch gekennzeichnet, daß man das Raney-Nickel oder Raney-Kobalt in der Reaktionszone magnetisch oder elektromagnetisch fixiert.

7. Verfahren nach den Ansprüchen 1 bis 6, dadurch gekennzeichnet, daß man in Sufe a eine Verweilzeit von 1 bis 30 Minuten einhält.

8. Verfahren nach den Ansprüchen 1 bis 7, dadurch gekennzeichnet, daß man eine Katalysatorbelastung von 0,2 bis 2,0 kg 5-Formylvaleriansäureester je Liter Katalysator und Stunde einhält.

9. Verfahren nach den Ansprüchen 1 bis 8, dadurch gekennzeichnet, daß man 5-Formylvaleriansäuremethylester, gelöst in Methanol, verwendet.

10. Verfahren nach den Ansprüchen 1 bis 9, dadurch gekennzeichnet, daß man in Stufe c die Umsetzung bei einer Temperatur von 80 bis 150° C durchführt.

## Claims

1. A process for the preparation of $\epsilon$-caprolactam by reacting a 5-formylvalerate with excess ammonia and hydrogen in the presence of a hydrogenation catalyst and in the presence of a solvent at elevated temperature and under superatmospheric pressure in the liquid phase, wherein

   a) the 5-formylvalerate is reacted with excess ammonia and hydrogen in the presence of an alkanol as the solvent and in the presence of a nickel or cobalt catalyst under a hydrogen partial pressure of from 5 to 1000 bar at from 40 to 130° C,

   b) ammonia, down to a content of 0.1 to 2% by weight, and hydrogen are separated off from the reaction mixture,

   c) the resulting reaction mixture is reacted with 1 to 20 times the amount by weight of water, based on the total content of 6-aminocaproate and $\epsilon$-caprolactam, at from 50 to 250° C with simultaneous removal of alkanols, and

   d) the reaction mixture thus obtained is heated to 150-370° C and $\epsilon$-caprolactam is obtained.

2. A process as claimed in claim 1, wherein the catalyst used is obtained by calcining a compound of the formula I

$$[(Mg_aNi(II)_bCO(II)_c)Al_2]CO_3(OH)_{16} \times 4H_2O$$

where a is an integer or decimal number from 0 to 4 and b and c are each an integer or a decimal number from 0 to 6, with the proviso that 2 (a + b + c) = 12, at from 200 to 600° C and then reducing the product with hydrogen at elevated temperatures.

3. A process as claimed in claim 1, wherein a nickel catalyst which contains from 30 to 60% by weight of finely divided nickel deposited on magnesium silicate is used.

4. A process as claimed in claims 1 to 3, wherein a 5-formylvalerate in solution with the alkanol and ammonia is passed over a fixed-bed catalyst by the liquid phase method.

5. A process as claimed in claim 1, wherein Ranay nickel or Raney colbalt is used in suspension.

6. A process as claimed in claims 1 and 5, wherein Raney nickel or Raney cobalt is fixed magnetically or electromagnetically in the reaction zone.

7. A process as claimed in claims 1 to 6, wherein a residence time of from 1 to 30 minutes is maintained in stage a.

8. A process as claimed in claims 1 to 7, wherein a space velocity of from 0.2 to 2.0 kg of 5-formylvalerate per liter of catalyst per hour is maintained.

9. A process as claimed in claims 1 to 8, wherein methyl 5-formylvalerate dissolved in methanol is used.

10. A process as claimed in claims 1 to 9, wherein the reaction is carried out at from 80 to 150° C in stage c.

## Revendications

1. Procédé de préparation d'$\epsilon$-caprolactame par réaction d'esters de l'acide 5-formylvalérianique avec de l'ammoniac en excès et de l'hydrogène, en présence de catalyseurs d'hydrogénation, avec utilisation simultanée de solvants, à température élevée et sous pression élevée, en phase liquide, caractérisé en ce que

   a) on fait réagir un ester de l'acide 5-formylvalérianique avec de l'ammoniac en excès et de l'hydrogène, avec emploi simultané d'alcanols comme solvants, en présence de catalyseurs au nickel ou au cobalt, sous une pression partielle d'hydrogène de 5 à 1000 bar et à une température de 40 à 130°C,
b) on élimine du mélange réactionnel obtenu l'ammoniac jusqu'à une teneur de 0,1 à 2% en poids et l'hydrogène,
c) on fait réagir le mélange réactionnel ainsi obtenu à une température de 50 à 250°C avec 1 à 20 fois la quantité en poids d'eau, par rapport à la teneur totale en ester d'acide 6-aminocaproïque et en ε-caprolactame, en séparant simultanément les alcanols et
d) on chauffe le mélange réactionnel ainsi obtenu à une température de 150 à 370°C et l'on obtient alors l'ε-caprolactame.

2. Procédé suivant la revendication 1, caractérisé en ce que l'on utilise des catalyseurs qui ont été préparés par calcination de composés de la formule I ci-après

$$[(Mg_aNi(II)_bCo(II)_c)Al_2]CO_3(OH)_{16} \times 4\,H_2O$$

dans laquelle a est mis pour des nombres entiers ou décimaux de 0 à 4 et b et c sont mis pour des nombres entiers ou décimaux de 0 à 6, avec cette condition que $2 \times (a + b + c) = 12$, à des températures de 200 à 600°C, et par réduction subséquente par l'hydrogène à température élevée.

3. Procédé suivant la revendication 1, caractérisé en ce que l'on utilise des catalyseurs au nickel qui contiennent 30 à 60 % en poids de nickel précipité sous forme finement divisée sur silicate de magnésium.

4. Procédé suivant les revendications 1 à 3, caractérisé en ce que l'on fait passer l'ester d'acide 5-formylvalérianique en solution dans des alcanols et l'ammoniac en courant ascendant sur un lit fixe de catalyseur.

5. Procédé suivant la revendication 1, caractérisé en ce que l'on utilise du nickel de Raney ou du cobalt de Raney en opérant en suspension.

6. Procédé suivant les revendications 1 et 5, caractérisé en ce que l'on fixe le nickel de Raney ou le cobalt de Raney dans la zone de réaction magnétiquement ou électromagnétiquement.

7. Procédé suivant les revendications 1 à 6, caractérisé en ce que, dans l'étape a, on maintient un temps de séjour de 1 à 30 mn .

8. Procédé suivant les revendications 1 à 7, caractérisé en oe que l'on maintient une charge du catalyseur de 0,2 à 2,0 kg d'ester de l'acide 5-formylvalérianique par litre de catalyseur et par heure.

9. Procédé suivant les revendications 1 à 8, caractérisé en ce que l'on utilise du 5-formylvalérianate de méthyle dissous dans du méthanol.

10. Procédé suivant les revendications 1 à 9, caractérisé en ce que, dans l'étape c, on effectue la réaction à une température de 80 à 150°C.